# EUROPEAN PATENT APPLICATION

(11) **EP 4 807 678 A1**
(43) Date of publication of application: **16.09.2026**
(21) Application number: 25163215.4
(22) Date of filing: 12.03.2025
(51) Int. Cl.: G06T 7/73

(54) **TECHNIQUE FOR DETERMINING A MARKER POSITION IN MEDICAL IMAGE DATA**

(71) Applicant: Stryker European Operations Limited, Carrigtwohill, Co. Cork T45 HX08 (IE)
(72) Inventor: KÖPFF, Marvin, 79227 Wolfenweiler (DE); MANSTETTEN, Alexandra, 79106 Freiburg im Breisgau (DE); RIEGELSBERGER, Fabian, 79211 Denzlingen (DE); UMBDENSTOCK, Emeric, 00185 Roma (IT); GERST, Maximilian, 79098 Freiburg im Breisgau (DE)
(74) Representative: Röthinger, Rainer

(57) **Abstract**

A technique for determining a position of a marker in a coordinate system defined by medical image data is provided. The medical image data has been generated using a radiation-based medical imaging technique and is indicative of streak lines due to the marker being located in a radiation beam path. A method implementation of the technique comprises identifying streak lines in the medical image data and determining, based on the identified streak lines, a position of the marker in the coordinate system

## Description

### Technical Field

The present disclosure generally relates to the field of navigated surgery. In particular, a method for determining a position of a marker in a coordinate system defined by medical image data is presented. The marker position may be used for image data registration. The present disclosure can also be implemented as a computer program product, an apparatus or a system.

### Background

In navigated surgery, it is common to track the position of a surgical tool relative to a patient using, for example, a tracking camera. Based on the relative position between the surgical tool and the patient, a navigation system generates navigation instructions for a surgeon or robot handling the surgical tool. The navigation instructions may indicate how the surgical tool needs to be manipulated towards a target position (e.g., on or within a bone of the patient).

The target position is often defined in medical image data of the patient, such as Computer Tomography (CT) data, while the tracked position of the surgical tool relative to the patient is represented in tracking image data taken by the tracking camera. For this reason, the medical image data and the tracking image data have to be registered before the current tool position can be correlated with the target position and before navigation instructions can be generated. Evidently, navigation accuracy and, thus, quality of the surgical outcome depends on the registration accuracy.

Conventional image registration techniques are based on identifying markers of a first type (e.g., radiopaque markers) that are detectable in the medical image data and that have a predefined spatial relationship to markers of a second type (e.g., Light Emitting Diodes, LEDs) that can be identified by the tracking camera. In some implementations, the two marker types are supported by a single registration device configured to be at least partially located both in an imaging volume of the medical imaging device and in a field of view of the tracking camera.

For the markers to be detectable in the medical image data, they are placed in an imaging volume of the medical imaging device. As a further constraint for marker placement, the markers should not obstruct the surgical procedure. Therefore, the markers are often placed on a patient's skin. However, in case the medical imaging device has only a small imaging volume (e.g., to reduce the patient's radiation exposure) or in case of an obese patient (i.e., when the markers on the skin have a large distance to an internal anatomical feature to be imaged), one or more of the markers on a patient's skin may lie outside the imaging volume. As a result, the problem arises that markers are not included in the medical image data, so their positions cannot be determined in a coordinate system defined by the medical image data. Consequently, the medical image data can in such cases not, or not accurately, be registered with tracking image data.

### Summary

There is a need for a technique for determining a position of a marker in a coordinate system defined by medical image data. In particular, there is need for determining the marker position even if the marker is located outside an imaging volume of a medical imaging device.

According to a first aspect, a computer-implemented method for determining a position of a marker in a first coordinate system defined by medical image data is provided. The medical image data has been generated using a radiation-based medical imaging technique and is indicative of streak lines due to the marker being located in a radiation beam path. The method comprises identifying the streak lines in the medical image data and determining, based on the identified streak lines, a position of the marker in the first coordinate system.

The medical image data may spatially be indicated by coordinates in the first coordinate system (e.g., the medical image data may define the first coordinate system by spanning the first coordinate system). The medical image data may have been generated using a medical imaging device. The medical imaging device may define an imaging volume (e.g., by one or more of an adjustable configuration parameter of the device, a mechanical device setup, and so on). The imaging volume may be adjustable. The marker may be located outside the imaging volume. If multiple markers are provided, they may all be located outside the imaging volume, or one or more may be located inside the imaging volume.

In some variants, the marker may be located in the imaging volume. In such variants, the marker position could independently be determined in a conventional manner and verified based on the streak lines identified in the medical imaging data.

The medical imaging device may comprise a radiation source, i.e. a radiation emitter, and a radiation detector located opposite to and spaced apart from the radiation source. The radiation beam path may be defined to extend between the emitter and the detector. The imaging volume may be defined to be located in the radiation beam path between the emitter and the detector. In some variants, the emitter and the detector may be moveable relative to the imaging volume.

An object to be imaged, e.g., an anatomical object such as a patient's spine, may be positioned in the imaging volume. The medical image data may be indicative of the object positioned in the imaging volume. In some variants, the marker may be located between the radiation emitter and the object that is to be imaged.

The marker, or a device carrying the marker (e.g., a registration device), may be loosely placed or attached to a skin of a patient. The imaging volume may be dimensioned so that the anatomical object to be imaged can be located in the imaging volume. The marker on the skin of the patient may be located outside the imaging volume.

The marker may be at least in part radiopaque so that the radiation-based (e.g., X-ray-based) medical imaging technique generates a corresponding artifact in the medical imaging data. For example, the marker may comprise a radiopaque portion and an optically detectable portion so that the marker may be identified in medical image data generated by the medical imaging device and in tracking image data generated by an optical tracking system, e.g., by a tracking camera of such a system. The radiopaque portion and the optically detectable portion of the marker may have a fixed spatial relation. As an example, they may essentially be co-located (e.g., an LED may constitute the optically detectable marker portion and a soldering spot of the LED may constitute the radiopaque marker portion). Multiple such markers may be supported by a single registration device. In other implementations, the registration device may comprise one or more, in particular three or more radiopaque markers (such as small metal balls) and one or more, in particular three or more optically detectable markers (such as LEDs or reflective spheres). The markers may have a predefined spatial relationship to each other that can be exploited for registering the medical image data and the tracking image data.

In some variants, the medical image data may comprise one or more 2-dimensional image data slices. In other variants, the medical image date may comprise 3-dimensional image data. In the latter case, the method may further comprise generating one or more 2-dimensional image data slices from the 3-dimensional image data. Each of the received or generated slices may be indicative of one or more streak lines. The position of the marker in the first coordinate system may be determined based on any of the slices. Using multiple of the slices for identifying streak lines may increase the accuracy of the determination of the position of the marker in the first coordinate system. For example, using multiple slices may allow a compensation of image data loss effects due to a limited resolution of a medical imaging device (e.g., due to the partial volume effect).

In some implementations, determining the position of the marker may comprise determining a respective intersection defined by the identified streak lines. For example, one or more of such intersections may be determined for each of the one or more 2-dimensional image data slices. Determining the position of the marker may further comprise determining an averaged intersection based on the respective intersections. The averaged intersection may define the marker position in the first coordinate system.

In some variants, the method may further comprise determining the position of the marker in the first coordinate system based on the determined one or more intersections and predetermined information. The predetermined information may be indicative of at least one of (i) a dedicated position of each of the one or more 2-dimensional image data slices in the first coordinate system, (ii) a dedicated orientation of each of the one or more 2-dimensional image data slices in the first coordinate system, and (iii) dedicated pixel spacings of each of the one or more 2-dimensional image data slices. The predetermined information may allow or at least facilitate identifying respective positions and orientations of the 2-dimensional image data slices in the 3-dimensional first coordinate system.

The predetermined information may comprise metadata associated with the one or more 2-dimensional image data slices. The metadata may comprise a dedicated slice number for each of the one or more 2-dimensional image data slices. Each dedicated slice number may be associated with two or more of (i) the dedicated position of a dedicated one of the 2-dimensional image data slices in the first coordinate system, (ii) the dedicated orientation of the dedicated one of the 2-dimensional image data slices in the first coordinate system, and (iii) the dedicated pixel spacings of the dedicated one of the one or more 2-dimensional image data slices. The dedicated slice number may facilitate retrieving the predetermined information associated with a dedicated slice of the one or more 2-dimensional image data slices.

The metadata may be provided or generated by the medical imaging device, for example when capturing the image data. The image data and, optionally, the metadata may be compliant with the Digital Imaging and Communications in Medicine, DICOM, standard.

The one or more 2-dimensional image data slices may comprise multiple 2-dimensional image data slices. In such variants, the method may further comprise generating a 3-dimensional model from the multiple 2-dimensional image data slices with the respective determined intersections. Moreover, the method may comprise determining, based on the generated 3-dimensional model, the position of the marker in the first coordinate system. Determining the marker position based on the generated 3-dimensional model may comprise weighting the respective determined intersections from the 2-dimensional image data slices based on at least one of (i) a number of identified streak lines contributing to the intersection and (ii) a number of different directions of the identified streak lines contributing to the intersection.

Multiple markers may be located in the radiation beam path. In such variants, the method may comprise determining multiple intersections defined by the identified streak lines and associating each of the intersections with a respective one of the multiple markers (e.g., by grouping the streak lines and/or their intersections). Further, the method may comprise determining a respective position in the first coordinate system for each of the multiple markers associated with one of the intersections. The method may further comprise defining groups of identified streak lines, each group being associated with a respective one of the multiple markers. Determining a respective intersection based only on the streak lines forming a part of a defined group of identified streak lines may improve the accuracy of the determination and may reduce computing resources needed for the determination of the respective intersections.

When multiple markers are located in the radiation beam path, the method may comprise determining at least some of the respective marker positions based on auxiliary information. The auxiliary information may comprise optical tracking data indicative of spatial relations between multiple markers, i.e., indicative of positions of multiple markers relative to each other. Additionally or alternatively, the auxiliary information may comprise a predetermined template (e.g., in the form of vectors, coordinates or distances) indicative of the spatial relations between multiple markers. As a result, computation resources needed for the determination of at least some of the marker positions may be reduced. In some variants, the respective positions determined via the streak lines may be compared to positions determined based at least in part on the auxiliary data. Consequently, the accuracy of the determined positions may be increased.

When the respective position in the first coordinate system has been determined for at least three of the multiple markers (with at least one of the positions being determined based on the streak lines), the method may further comprise an image registration procedure. The image registration procedure may result in transformation information that allows to register the first coordinate system with a second coordinate system.

An exemplary image registration procedure may comprise receiving (e.g., optical or electromagnetic) tracking data indicative of positions of the at least three of the multiple markers, or of at least three further markers (e.g., optical markers such as LEDs or reflective spheres in case of an optical tracking system or electromagnetic markers such as one or more coils in case of an electromagnetic tracking system with an electromagnetic field sensor), in the second coordinate system. The at least three further markers have a predetermined spatial relationship to the at least three of the multiple markers. The registration procedure may further comprise determining, based on the determined positions of the at least three of the multiple markers in the first coordinate system and the tracking data, a registration between the first coordinate system and the second coordinate system. As a result of the registration, both the medical image data and the tracking data may be rooted in a common coordinate system, e.g., the first coordinate system or the second coordinate system. The registration may be automatically trigged when the respective position in the first coordinate system has been determined for at least three of the multiple markers and the tracking data has been received. In case of an optical tracking system, the tracking data may be image data (also called tracking image data herein). In case of an electromagnetic tracking system, the tracking data may be indicative of coil positions (e.g., in the second coordinate system).

The second coordinate system may be associated with one of a tracker attached to the patient, a tracker attached to the medical imaging device, or a predetermined point of a tracking camera. In some implementations, the second coordinate system may be defined by the tracking image data (e.g., the tracking image data may spatially be indicated by coordinates in the second coordinate system). The tracking image data may have been generated by a tracking camera capable of capturing optically detectably markers (e.g., in the infrared spectrum).

In some variants, the method may further comprise defining a (virtual) tracker based on multiple of the determined marker positions in the first or second coordinate system. For example, a tracker may be defined based on two or more of the determined marker positions, in particular based on all of the determined marker positions. For example, a tracker trackable in five degrees of freedom, DOF, may be defined based on two of the determined marker positions. In addition, or as an alternative, a tracker trackable in six DOF may be defined based on three or more of the determined marker positions.

The medical image data may be or may include CT image data. The imaging volume may be a scan volume of a CT scanner. In other implementations, the imaging volume may be defined by cone beam intersections of a cone beam CT device.

In some variants, the method may be implemented to use machine learning for executing one or more of the method steps. For example, the method may be implemented using a neural network, e.g., a deep neural network or a convolutional neural network.

The method may further comprise processing the medical image data to facilitate at least one of the determination of the marker position and the identification of the streak lines. Processing the medical image data may comprise enhancing a contrast of the image data to improve (e.g., automatic) detectability of the streak lines. The contrast may be enhanced using known image processing techniques, e.g., Contrast Limited Adaptive Histogram Equalization (CLAHE). Additionally or alternatively, processing the medical image data may comprise filtering the image data to reduce noise therein. The noise may be reduced based on known image processing techniques, e.g., by applying an Anisotropic Distribution Filter.

Processing the medical image data may comprise determining pixel intensity gradients and identifying the streak lines based on the pixel intensity gradients. Determining the pixel intensity gradients may comprise determining a dedicated intensity for each pixel of the medical image data. Further, the intensity gradients may be defined based on a comparison of the dedicated intensities of adjacent pixels. For example, the gradients may be determined based on the magnitudes of differences between the dedicated intensities of adjacent pixels. In the following, an example for image data processing is discussed in greater detail for two-dimensional image data. However, three-dimensional image data could be processed analogously.

An intensity gradient at a given pixel of a 2-dimensional image may be defined based on a tensor ${J}_{xy} = \left[\begin{matrix}{I}_{x}^{2} & {I}_{x} {I}_{y} \\ {I}_{x} {I}_{y} & {I}_{y}^{2}\end{matrix}\right] ,$ with Iₓ being a gradient in the direction of an x-axis of the first coordinate system defined by the medical image data and I_{y} being a gradient in the direction of a y-axis of the first coordinate system defined by the medical image data. The Eigenvectors of the tensor may be indicative of a gradient orientation and the Eigenvalues may be indicative of a magnitude of the gradient, i.e., the amount of a change of an intensity value.

The method may further comprise defining intensity vectors based on the intensity gradients and grouping the vectors, e.g., based on their orientation and position, and, optionally, based on their intensity value. Grouping the vectors may comprise defining a new vector, i.e., a group vector, based on multiple intensity vectors. The intensity vectors may be defined based on the Eigenvectors and Eigenvalues of the tensor. For example, an intensity vector may be defined as ${v}_{xyθm} = \left[\begin{matrix}x \\ y \\ θ \\ m\end{matrix}\right] ,$ with x and y defining a position of a vector origin in the first coordinate system defined by the medical image data, θ defining an orientation of the vector with respect to the x-axis and m defining an intensity value at the origin position.

For grouping the defined intensity vectors, i.e. for determining a group vector, the intensity vectors for which the intensity value m is smaller than a threshold may be labeled as noise. Further, the orientation θ may be split into ranges (e.g., Δθ = 10°) and vectors not identified as noise may be grouped based on the defined ranges of the orientation. Still further, a number of neighboring pixels, i.e., an area of the image data may be defined for which the intensity vectors are to be grouped. The grouped intensity vectors, e.g., the resulting group vector, may indicate a dominant orientation of gradients in the defined area of the image data. The dominant orientation may indicate edges of streak lines and/or anatomical objects indicated in the medical image data since such edges generally result in high gradients in the medical image data. The number of neighboring pixels, i.e., the size of the area in which pixels are to be grouped may be based on a predetermined value. The predetermined value may be based on user input. Based on the grouped intensity vectors, i.e., based on the intensity gradients indicated by the grouped intensity vectors, group lines indicating the intensity changes in the medical image data may be defined. The group lines may indicate the streak lines, i.e., intensity changes due to image artifacts generated due to the radiopaque marker, as well as intensity changes in the medical image data due to different anatomical objects (e.g., different bones).

In some variants, at least one of the tensor according to equation (1) and the vector groups (and/or the dominant orientations) may be determined multiple times. The method may comprise determining an average value for at least one of the tensor, the vector groups, and the dominant orientations based on the multiple determinations.

To identify the streak lines indicated by the group lines, the method may further comprise filtering the processed medical image data based on an estimated location of a marker relative to an object that is shown in the medical image data. In some variants, the filtering may comprise filtering the group lines based on respective estimated positions of one or more markers. For example, all group line portions and/or intersections located in the patient may be filtered out due to the one or more markers being known to be located on the skin of the patient, i.e., outside the patient (or the image volume). Further intersections may be filtered out based on a known relative position between markers.

Further, the method may comprise determining a group line density. Determining the group line density may comprise determining intersections of the remaining group lines and determining the density of group lines for the intersections. Due to the majority of the group lines being associated with one or more anatomical objects of the patient indicated in the medical image data having been filtered out, intersections with a high group line density, e.g., compared to a total number of group lines or a predetermined density of group lines, may be identified as streak line intersections. In other words, high density points may be identified as streak line intersections, the associated group lines as streak lines, and the associated density of lines as streak line density. In case of a single marker only the highest density point, hdp, of group lines may be determined.

The method may further comprise determining more detailed intensity vectors for a region of predetermined size around at least one of the high density points, i.e., for a part of the medical image data indicative of the at least one of the high density points. The more detailed intensity vectors may be determined by defining vector groups with fewer vectors per group compared to the previous grouping, i.e., the grouping for the received medical image data. As a result, the streak lines associated with a high density point and their intersections in the predetermined area may be determined in more detail (i.e., with in increased accuracy).

The predetermined size of the area may have a default size or may be defined based on received user input. In some variants, the method may comprise receiving further user input and adapting the size of the area based on the received further user input.

In some variants, the method may comprise determining clusters of group vectors and determining a centre of mass of each cluster. The clusters may be defined based on the locations of origins of the group vectors in the first coordinate system defined by medical image data, e.g., to associate each cluster with an anatomy of a patient or a marker positioned on the skin of the patient. The centers of mass of the respective clusters may be determined based on an intersection of group lines defined based on the group vectors of the respective cluster.

The method may further comprise fitting the respective centers of mass to an estimated position or to estimated positions of one or more markers. The position(s) may be estimated based on the one or more markers being located outside the patient (or the image volume) and/or based on known spatial relations between at least some of the markers. The method may further comprise identifying the group lines of a cluster as streak lines based on the fitting results. For example, the group lines associated with a cluster of group vectors comprising the center of mass with the best fit to an estimated marker position may be identified as streak lines. In some variants, the streak lines may be identified based on the determination of a density map and/or the center of mass fits as described herein.

In some variants, processing the medical image data may comprise highlighting regions in the image data based on a degree of anisotropy. For example, a degree of anisotropy may be determined for each pixel based on the tensor defined in equation (1), and pixels may be highlighted based on their relative degree of anisotropy (e.g., compared to the degree of anisotropy of the other pixels of the medical image data). In particular, pixels with a high relative degree of anisotropy may be highlighted compared to pixels with a low relative degree of anisotropy.

Each of the image data processing steps described herein may increase detectability of the streak lines and, thus, accuracy of the determination of the marker position(s).

According to a second aspect, a computer program product is provided. The computer program product comprises instructions that, when executed on at least one processor, cause the at least one processor to carry out the method according to first aspect. The computer program product may be stored in a computer-readable storage medium.

According to a third aspect, an apparatus for determining a position of a marker in a first coordinate system is provided. The first coordinate system is defined by medical image data generated using a radiation-based medical imaging technique. The medical image data is indicative of streak lines due to the marker being located in a radiation beam path. The apparatus comprises a processor configured to identify streak lines in the medical image data. The processor is further configured to determine, based on the identified streak lines, a position of the marker in the first coordinate system.

The apparatus may be any computing device comprising at least one processor, e.g., a personal computer or a server. The apparatus may comprise a memory. The memory may be configured to store the computer program product according to the second aspect.

In some variants, the apparatus is configured to perform the method according to the first aspect.

According to a fourth aspect, a system is provided. The system comprises the apparatus according to the third aspect. In one variant, the system further comprises a medical imaging device configured to generate medical image data using a radiation-based medical imaging technique. In this or another variant, the system further comprises a registration device. The registration device may comprise one or more of the markers presented herein. As an example, the registration device may comprise at least one marker with an optically detectable portion and a radiopaque portion. Each such marker may further comprise an adhesive portion for releasably attaching the marker to an object, e.g., a skin of a patient. Multiple such markers may independently be attached around an incision for accessing an anatomical object (e.g., a spine) of the patient. In another example, multiple such markers are integrated in a frame that can jointly be attached (e.g., via an adhesive) around the incision. In a still further example, the registration device comprises one or more radiopaque markers and one or more optically detectable markers in a predefined spatial relationship to the one or more radiopaque markers. The predefined spatial relationship may be indicated by coordinates or vectors.

### Brief Description of the Drawings

Further details, advantages and aspects of the present disclosure will become apparent from the following embodiments taken in conjunction with the drawings, wherein:
- Fig. 1: shows an embodiment of a system for determining a marker position;
- Fig. 2A: shows an exemplary marker of Fig. 1 in more detail;
- Fig. 2B: shows six markers of Fig. 2 attached to a skin of a patient;
- Fig. 3: shows the system of Fig. 1 with a different patient being imaged;
- Fig. 4: shows a flow diagram of a method for determining a marker position;
- Fig. 5: shows a schematic representation of multiple 2-dimensional image data slices arranged in a 3-dimensional coordinate system;
- Fig. 6: shows an exemplary medical image illustrating streak lines generated by a radiopaque object located in a radiation beam path; and
- Figs. 7A-7H: show processed medical image data after different processing steps.

### Detailed Description

In the following description of exemplary embodiments, the same reference numerals are used to denote the same or similar components.

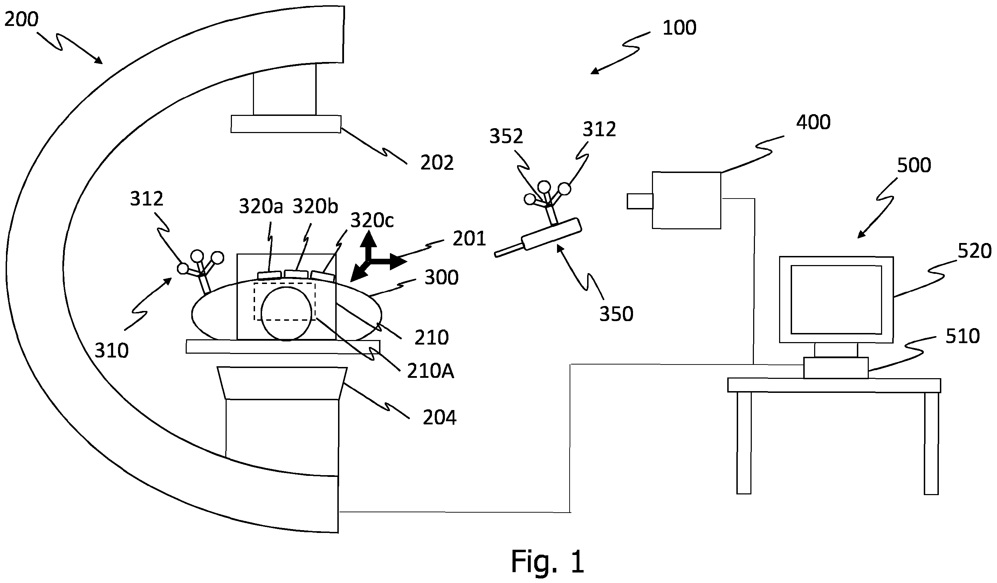

Fig. 1 shows an embodiment of a system 100 according to the present disclosure. The system 100 comprises a radiation-based medical imaging device 200. The medical imaging device 200 is configured to generate at least one of 2-dimensional and 3-dimensional medical image data defining an image coordinate system 201. In some implementations, the medical image data may be conform to the DICOM standard. In such implementations, the coordinate system 201 may be a DICOM reference coordinate system (RCS) or similar coordinate system.

The exemplary medical imaging device 200 of Fig. 1 is realized as a C-arm. Other suitable imaging devices are contemplated (e.g., realized as an O-arm or gantry).

As illustrated in Fig. 1, the medical imaging device 200 comprises an emitter 202 configured to emit radiation, typically X-rays, and a detector 204 configured to detect the radiation emitted by the emitter 202. The space between the emitter 202 and the detector 204 defines a radiation beam path. The radiation beam path extends through an imaging volume 210 of the medical imaging device 200. The imaging volume 210 is represented in the medical image data generated with the medical imaging device 200. At least a part of an object to be imaged, in this example one or more anatomical objects (e.g., a spine) of a patient 300, is located in the imaging volume 210. The imaging volume 210 may be an (optionally adjustable) configuration parameter of the medical imaging device 200.

In some variants, the imaging volume 210 may be defined by a manufacturer of the medical image device 200. In certain implementations, the manufacturer may define the imaging volume 210 based on a number of X-ray traces per volume unit in a region between the emitter 202 and the detector 204. The number of X-ray traces per volume unit may be indicative of a number of 2-dimensional images that can be generated per volume unit by the imaging device 200 and, thus, of an accuracy of a 3-dimensional volume that may be generated (i.e., reconstructed) based on the 2-dimensional images. The actual imaging volume 210 may include those volume units between the emitter 202 and the detector 204 that include a sufficient number of X-ray traces to reconstruct the imaging volume with a sufficient accuracy. As such, a minimum threshold for the number of X-ray traces per volume unit may be defined, that allows generation of a predetermined number of 2-dimensional images (i.e., that allows to reconstruct the 3-dimensional image volume 210 with a predetermined accuracy). In some variants the imaging volume 210 is calculated by a software. The actual size, location and orientation of the imaging volume 210 can be derived in a calibration phase by the manufacturer, and it may be re-calibrated at certain intervals. Moreover, a software may allow a user to choose between different imaging volumes 210, e.g., between two or more sizes for the imaging volume 210.

The system 100 of Fig. 1 further comprises a patient tracker 310 attached to the patient 300. The patient tracker 310 may be attached to a skin surface of the patient 300 (see, e.g., the non-invasive CranialMask^{®} or SpineMask^{®} tracker of Stryker Corp.) or to the anatomical object of interest (e.g., a vertebra bone). The exemplary patient tracker 310 illustrated in Fig. 1 is a conventional optical tracker with multiple optically detectable markers, such as reflective spheres 312 or LEDs. A patient coordinate system may be defined based on (e.g., may be rooted in) the patient tracker 310.

Fig. 1 also illustrates a surgical tool 350 (e.g., a drill or screwdriver) with an associated tool tracker 352. The surgical tool 350 may need to be guided by navigation instructions (e.g., along a planned target trajectory) towards the anatomical object of interest.

The system 100 further comprises multiple markers 320a, 320b, 320c individually attached to the patient 300, in particular to the skin of the patient 300. In the example of Fig. 1, the markers 320a, 320b, 320c are located in the imaging volume 210.

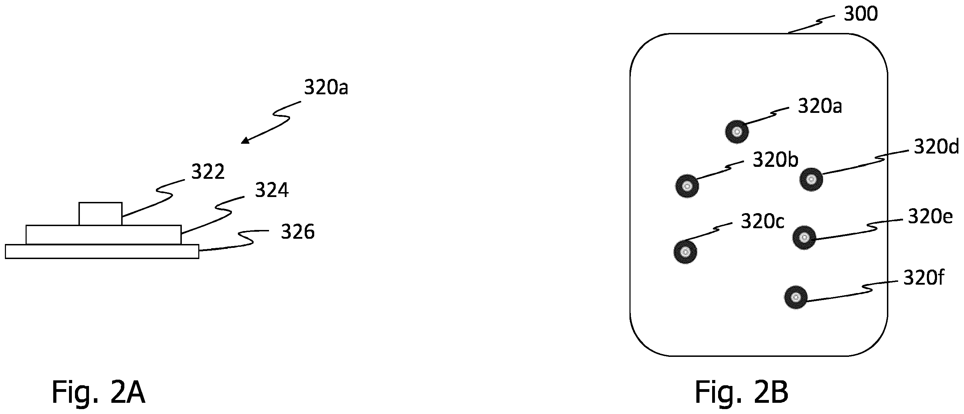

With reference to the schematic illustration of Fig. 2A, each of the markers 320a, 320b, 320c comprises an optically detectable (and thus trackable) portion 322, a radiopaque portion 324 and an adhesive portion 326. These portions 322, 324, 326 may stacked one above the other or have a nested configuration.

In an exemplary realization, the optically detectable portion 322 is an active light emitter, such as an LED, or simply a passive marking (e.g., a reflective sphere or a color spot). In some implementations, the optically detectable portions 322 of the markers 320a-320c can jointly form a patient tracker (similar to the SpineMask^{®} or the CranialMask^{®}, in which case no dedicated patient tracker 312 as illustrated in Fig. 1 will be needed). In case electromagnetic tracking is desired, the optically detectable portion 322 could be replaced by one or more coils or other electromagnetic sensors.

With continued reference to Fig. 2A, the radiopaque portion 324 of the marker 320a may comprise a metal ball embedded in a radiolucent substrate. As an example, the metal ball of the radiopaque portion 324 may substantially be arranged in a center of, and thus be co-located with, a color spot of the optically detectable portion 322. In other variants, the optically detectable portion 322 can be a reflective sphere and the metal ball of the radiopaque portion 324 can be located in a center of the reflective sphere. The adhesive portion 326 may be realized as a flat substrate with an adhesive on its side facing away from the radiopaque portion 324.

While only three markers 320a, 320b, 320c are shown in Fig. 1, any number of markers 320a, 320b, 320c may be attached to the patient 300. An exemplary configuration of six markers 320a, 320b, 320c, 320d, 320e, 320f arranged on a skin of the patient 300 in a freely selected spatial relation to each other (e.g., around an incision) is shown in Fig. 2B. It should be noted that the function of the individual markers 320a-320f illustrated in Fig. 2B could also be realized by the Stryker SpineMask^{®} tracker (or similar tracker) that carries a number of LEDs. Each of these LEDs constitutes an optically detectable marker portion, and an associated soldering spot for connecting the LED to a printed circuit realizes a radiopaque marker portion substantially co-located with the LED.

In some variants, a first number of radiopaque markers and a second number of optical or electromagnetic markers that are spaced apart from the radiopaque markers may be arranged on the skin of the patient 300. In some variants, each of the first number of radiopaque markers may be arranged in a predetermined spatial relation to at least one of the second number of optical or electromagnetic markers. The first number of markers may be different from the second number of markers.

Returning to Fig. 1, the system 100 comprises a tracking camera 400 configured to optically track the patient tracker 310, the tool tracker 350 and the optically detectable portions 322 of the markers 320a, 320b, 320c, and to generate tracking image data indicative of these tracked devices 310, 350/352, 320a, 320b, 320c. The tracking camera 400 may be realized as a stereo camera capable of operating in the IR and/or visible spectrum.

In other realizations, electromagnetic tracking may be used. In such realizations, a field generator may be provided and the tracked devices may carry field sensors (e.g., coils). The resulting tracking data will be indicative of positions of the field sensors in a coordinate system rooted, for example, in the field generator.

The system 100 illustrated in Fig. 1 comprises a computing device 500 configured to receive and process medical image data from the medical imaging device 200 and tracking image data from the tracking camera 400. The computing device is also configured to perform an image registration procedure such that the medical image data and the tracking image data can be correlated in a common coordinate system that may, for example, be rooted in the patient tracker 312 or in the tracking camera 400.

In some variants, the computing device 500 is further configured to generate navigation instructions from the processed image data and from surgical planning data. The surgical planning data may define a target location of the surgical tool 350 (or of an implant) relative to an anatomical object represented in the medical image data. Additionally, or in the alternative, the surgical planning data may define a target trajectory of the surgical tool 350 relative to an anatomical object represented in the medical image data.

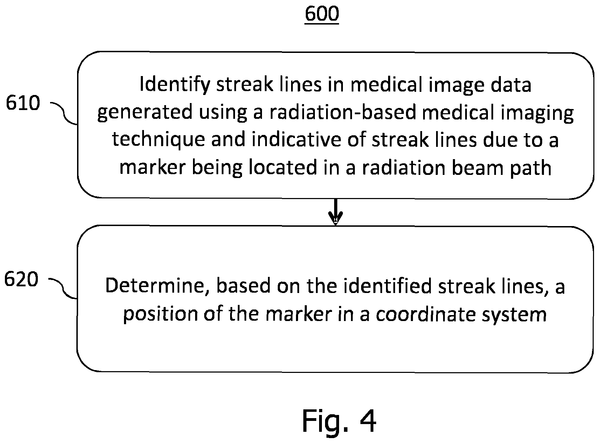

The computing device 500 illustrated in Fig. 1 comprises a processor 510 configured to determine a position of a marker 320a, 320b, 320c in the image coordinate system 201 (as will be discussed in detail below with reference to Figs. 4 to 7H). The processor 510 is further configured to perform an image registration procedure based on the determined marker positions and to generate navigation instructions. The computing device 500 comprises a display 520 configured to display image data generated or processed by the processor 510. In a surgical navigation scenario, such image data comprise navigation instructions visualized by the display 520. The computing device 500 may further comprise at least one user input device (not shown), such as a keyboard and a computer mouse.

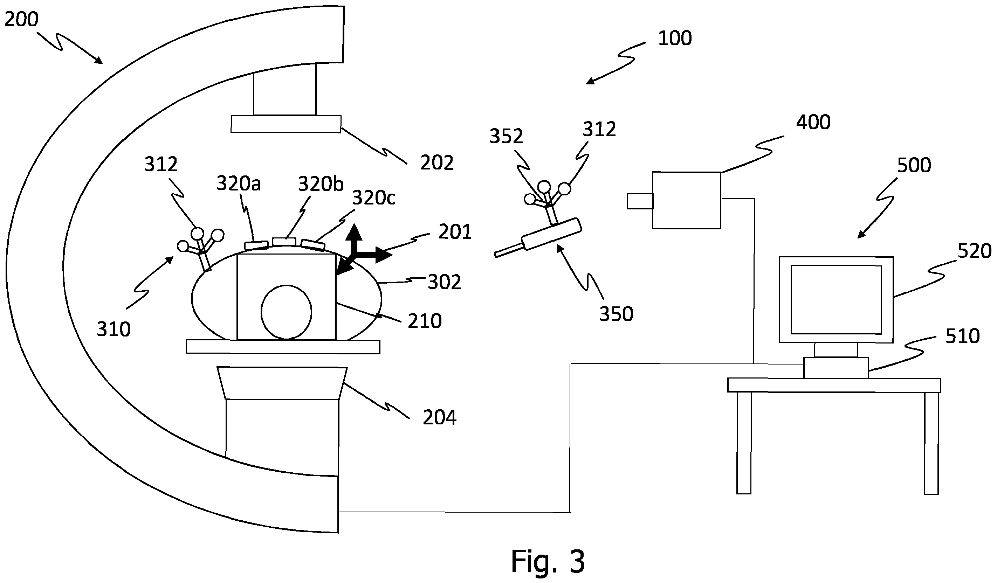

Fig. 1 shows in dashed lines a smaller imaging volume 210A in an alternative configuration of the medical imaging device 200. Selecting a smaller imaging volume may be desirable to reduce radiation exposure for the patient 300. Fig. 3 shows the system of Fig. 1, wherein a different patient 302 is located in the medical imaging device 200. The patient 302 shown in Fig. 3 is bigger than the patient 300 of Fig. 1. As a result of the smaller imaging volume 210A of Fig. 1 or the bigger patient 300 of Fig. 3, the markers 320a, 320b, 320c are all (or at least one or more of them) located outside the respective imaging volume 210, 210A. This also means that the markers 320a, 320b, 320c will not all be included in the medical image data, and that their coordinates in the image coordinate system 201 (that, as a coordinate system, also extends beyond the actual medical image data) cannot be determined using conventional approaches. Therefore, image registration procedures and other procedures that rely on the marker positions in the image coordinate system 201 lack the required information.

Fig. 4 shows a flow diagram 600 of a method for determining a position of one or more of the markers 320a, 320b, 320c in the image coordinate system 201. This method allows to determine a marker position also in cases in which a particular marker 320a, 320b, 320c is located outside the imaging volume 210, 210A. Moreover, the method allows to verify the marker position of a particular marker 320a, 320b, 320c located inside the imaging volume 210, 210A

With reference to Fig. 4, the method comprises a step 610 of identifying streak lines in medical image data generated by the medical imaging device 200 of Figs. 1 or 3. Streak lines are generally undesired image artifacts in medical image data. These image artifacts may be caused by radiopaque, e.g., metallic, objects (in particular the markers 320a, 320b, 320c) located in the radiation beam path between the radiation emitter 202 and the radiation detector 204. The streak lines may generally appear as alternating bright and dark streaks with generally linear shapes caused by at least one of photon starvation, beam herding and scattering.

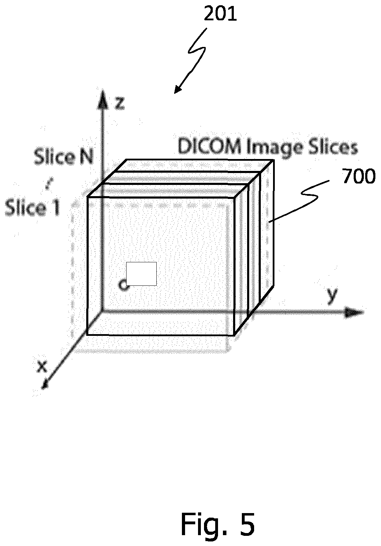

The medical image data generated by the medical imaging device 200 (e.g., in the DICOM format) may comprise multiple 2-dimensional image slices 700 as schematically shown in Fig. 5. Each of the slices 700 shown in Fig. 5 is associated with metadata, such as a dedicated slice number 1 to N. Each dedicated slice number is associated with a position and orientation of a dedicated slice 700 in the image coordinate system 201. The slice number thus allows fast and easy determination of a position and orientation of a dedicated slice 700 of the multiple slices 700 in the image coordinate system 201. Further, construction of a 3-dimensional model based on multiple neighboring slices 700 is facilitated. It is to be noted that while Fig. 5 illustrates the construction of a 3-dimensional model from slices 700, the 3-dimensional model will be constructed differently if a different imaging modality is used. For cone beam CT, for example, the 3-dimensional model may be constructed from 2-dimensional images using ray-tracing procedures.

Returning to Fig. 5, the streak lines may be identified either in the 2-dimensional slices 700 or the 3-dimensional model constructed therefrom. The more slices 700 being used for identifying streaks in step 610, the higher the positional accuracy due to additional data points being available for streak identification. However, using more slices 700 may require more computational resources. Depending on the available computational resources of the computing device 500, a "sweet spot" (i.e., a preferred number of slices 700 to be analyzed) may be determined.

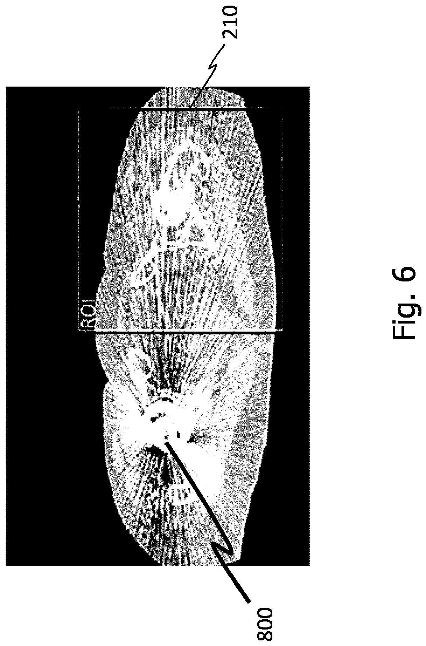

For illustration purposes, Figs. 6 shows an exemplary 2-dimensional CT image indicative of streak lines generated by a radiopaque object 800 (such as one of the markers 320a, 320b, 320c) located in a beam path of the imaging device 200. As can be seen in Fig. 6, a position of the object 800 in a coordinate system associated with the shown region of interest (ROI; for example a cross-section of the imaging volume 210) can be determined (see step 620 of Fig. 4) based on an intersection of the streak lines outside the ROI and, thus, by extrapolation, even if the object 800 is not located in the ROI (i.e., if the object 800 is located outside the imaging volume 210, 210A). Ideally, this intersection is a point. In practice, based on the size of the object 800, the intersection defines a certain area, as illustrated in Fig. 6. In such a case, the object position can be determined using mathematical averaging approaches such as weighting individual intersections of two streak lines or determining a center of gravity of an area spanned by the individual intersections.

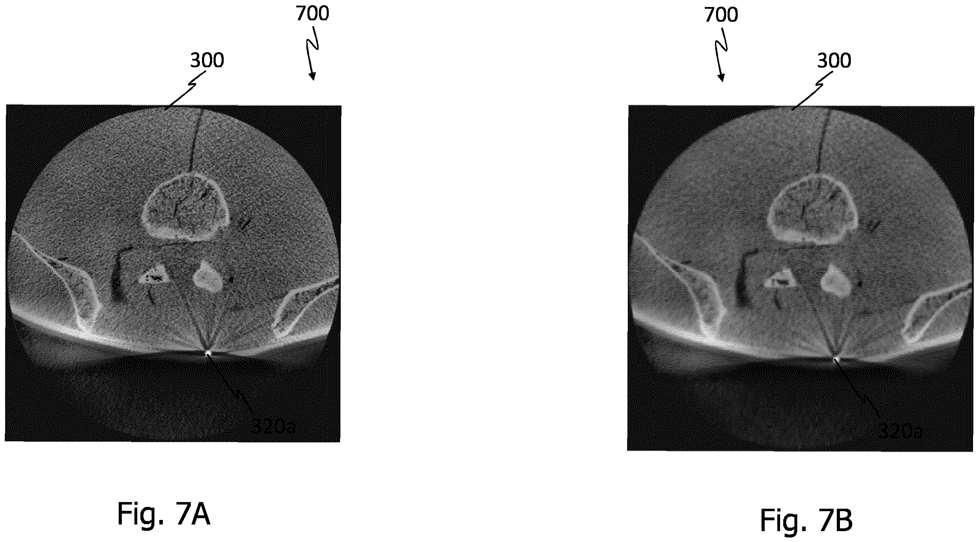

The step 610 of identifying streak lines may comprise (pre-)processing a dedicated slice 700 generated by and received as medical image data from the medical imaging device 200. Processing the received medical image data may improve the accuracy of streak line identification since the automatic detectability (i.e., the "visibility") of streak lines and of their intersections can be improved. Examples of different states of a processed dedicated slice 700 (i.e., of different sub-steps of the step 610) are shown in Figs. 7A to 7H. While for clarity purposes only one marker 320a is shown in Figs. 7A to 7H, the method may be performed for images indicative of multiple markers 320a, 320b, 320c analogously.

For illustration purposes, Fig. 7A shows a medical image data slice 700 indicative of a patient 300 and a marker 320a attached to the skin of the patient 300. It is to be noted that the marker 320A could also lie outside image data slice 700 and, thus, the imaging volume 210. In such a case, the streak lines would need to be extrapolated to their intersection(s) outside the imaging volume 210.

The image data slice 700 of Fig. 7A is filtered using an anisotropic distribution filter for multiple filtering iterations. The filtered slice 700 shown in Fig. 7B is the result of the 10^{th} iteration. As can be seen, image noise has been reduced, resulting in a smoothed image, wherein anatomical objects of the patient 300 and the marker 320a as well as the artifacts generated due to the marker 320a, i.e., the streak lines, are conserved. It is to be noted that the streak lines would still be present even if the marker 320a was not in the imaging volume.

Based on the filtered slice 700 shown in Fig. 7B, an intensity gradient is determined for each pixel thereof. The intensity gradients are determined based on the following tensor (analogous to equation (1) explained herein and adapted to the example coordinate system 201 shown in Fig. 5): ${J}_{xyz} = \left[\begin{matrix}{I}_{xy}^{2} & {I}_{xy} {I}_{xz} \\ {I}_{xy} {I}_{xz} & {I}_{xz}^{2}\end{matrix}\right] ,$ with I_{y} being a gradient in the direction of a y-axis of the coordinate system 201 shown in Fig. 5, I_{z} being a gradient in the direction of a z-axis of the coordinate system 201 shown in Fig. 5, and x being the coordinate in the direction of an x-axis of the coordinate system 201 shown in Fig. 5. The x-axis coordinate is associated with the slice number, i.e., 1 to N.

The Eigenvalues of the tensor for a dedicated pixel, which indicate the gradient magnitude of the dedicated pixel, are determined for each of the pixels and used to further filter the slice 700 shown in Fig. 7B. Each pixel is enhanced based on its determined gradient magnitude.

Still further, a degree of anisotropy (i.e., a measure for the change of a physical property depending on the direction) is determined for each pixel of the enhanced image data, and the enhanced image data is further filtered based on the determined degrees of anisotropy.

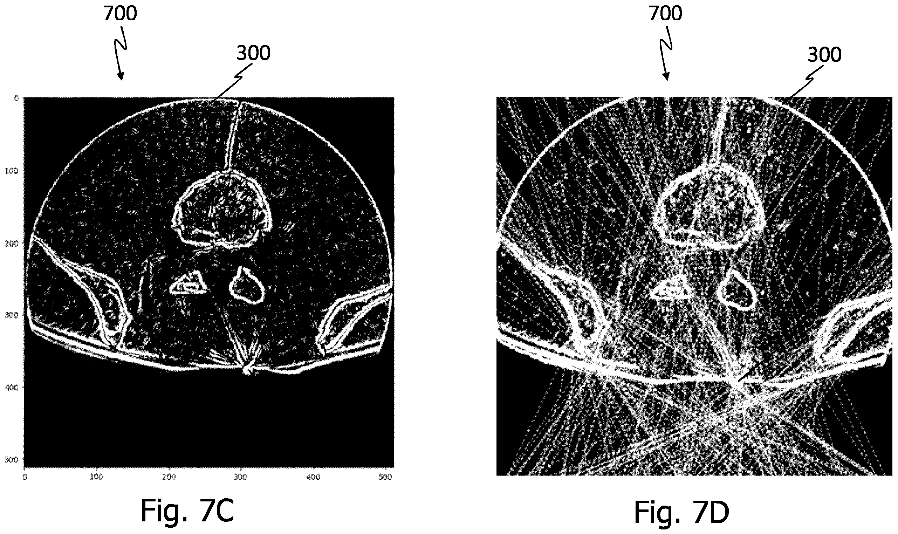

A result of the beforementioned image processing (i.e., filtering) steps is shown in Fig. 7C. The image artifacts, e.g., streak lines, and the image objects, i.e., the anatomical objects and the marker 320a, represented in the slice 700 are now more clearly discernable compared to the unprocessed slice 700 shown in Fig. 7A.

As can be seen from Fig. 7C, the aforementioned image processing steps are suited for highlighting streak lines since streak lines are generally indicated by alternating bright and dark image regions identifiable based on the intensity gradient and since the streak lines generally have a linear shape, i.e., a high degree of anisotropy. While in the shown example the filtering steps have been applied in a defined sequence, other sequences of the filtering steps may be used. Further, only one or more of the described filtering steps may be used. Still further, any of the described filtering steps or any combination thereof may be combined with other known image filtering steps.

Next, the tensor *J_{xyz}* is re-determined based on the processed slice 400 shown in Fig. 7C. Moreover, intensity vectors are determined for each pixel based on the re-determined tensor *J_{xyz},* i.e., based on the Eigenvalues and Eigenvectors of the re-determined tensor *J_{xyz}.* An intensity vector is defined as: ${v}_{xyzθm} = \left[\begin{matrix}x \\ y \\ z \\ θ \\ m\end{matrix}\right] ,$ with y and z defining a position of a vector origin in a plane at position x of the coordinate system 201 shown in Fig. 5, θ defining an orientation of the vector with respect to the y-axis of the coordinate system 201 shown in Fig. 5, and m defining an intensity value at the origin position.

Further, in the present example, the intensity vectors are filtered. In particular, all vectors with an intensity value m smaller than a threshold are labeled as noise and filtered out. Still further, the orientation θ is split into ranges, e.g., Δθ = 10°. Moreover, the slice 700 is split up in predetermined areas and group vectors are determined for each predetermined area based on the orientation ranges. In particular, the vectors that are associated with a pixel located in a predetermined area of the processed slice 700 and not filtered out are grouped based on the defined ranges of the orientation, i.e., a group vector representing an (optionally weighted) average of the grouped vectors is determined. The predetermined areas have been determined based on user input. Each of the predetermined areas comprises multiple pixels, e.g., 2, 3, 4, 9, 16 or more pixels. The size of the predetermined areas may vary for the slice 700, i.e., the slice 700 may be split up into areas of different sizes. For example, parts of the slice 700 of greater interest may be split into smaller areas, resulting in more detailed group vectors for the parts of greater interest.

Further, a group line is determined for each vector of the group vectors. Each group line is determined based on the orientation of a respective one of the group vectors. The processed slice 700 comprising determined group lines is shown in Fig. 7D.

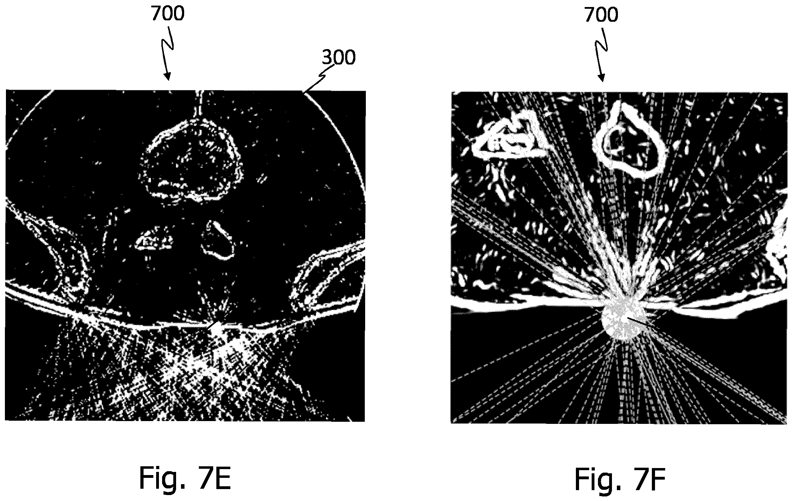

The slice 700 of Fig. 7D is then further processed by filtering the group lines. The group lines are filtered by filtering out all group line portions located in the patient 300, resulting in the slice 700 shown in Fig. 7E. In other variants, markers may be located within the patient. In such variants, the group lines may be filtered based on obtained additional information indicative of a (rough) marker position, e.g., known from marker placement.

Next, all intersections of the remaining group line portions are determined and a density map of the intersections is determined. Based on the density map an area with the highest density of intersections is determined. Due to the previously explained characteristics of streak lines (i.e., generally alternating dark and bright linearly shaped areas) and the accordingly chosen image processing steps, the group lines defining the highest density area of intersections can be identified as the streak lines, i.e., as the artifacts associated with the marker 320, 320A.

For a more detailed determination of the intersections in the highest density area, a cutout of the image slice 700 around the highest density area is further processed as shown in Fig. 7F. In particular, a new set of more detailed group lines is determined, i.e., group lines based on group vectors determined based on relatively small predetermined areas. The newly determined group lines, which can now reasonably be assumed to be streak lines, and intersections thereof are then determined, as shown in Fig. 7F.

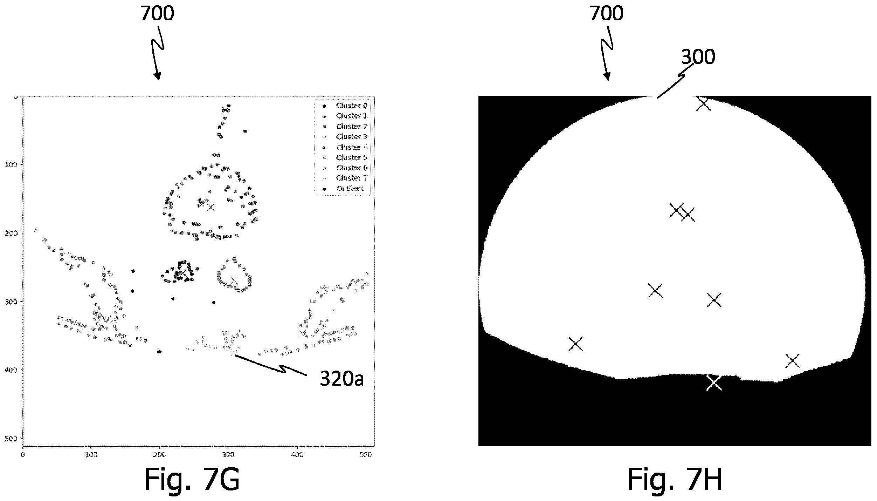

Further or other image processing steps not shown in Figs. 7A to 7F may be performed. For example, alternatively to filtering out the group line parts located within the patient 300 as shown in Fig. 7E, the determined group vectors may be clustered based on their positions within the slice 700, as shown in Fig. 7G. For each cluster of group vectors a centre of mass may be determined. The center of mass of each cluster may be defined as the highest density area of intersections of group lines defined by the group vectors of the cluster.

The determined centers of mass may be fitted to an estimated position of the marker 320a as indicated in Fig. 7H. The group lines defined based on the group vectors of the cluster with the center of mass that fits the best to the estimated position of the marker 320a are identified as streak lines caused by marker 320a. Further, the cutout shown in Fig. 7F may then be generated based on the best fitting center of mass as shown in Fig. 7H.

Returning to Fig. 4, the method comprises the step 620 of determining a position of the marker 320a in the image coordinate system 201 based on the identified streak lines, e.g., based on the intersections of the detailed streak lines determined in the cutout shown in Fig. 7F. For example, an average intersection location may be determined and identified as the center point of the marker 320a, as indicated by a cross in Fig. 7F. The location of this cross in the image coordinate system 201 (see Fig. 5) identifies the position of the marker 320a. It will be readily apparent that this marker position could also be determined if the marker 320a was located outside the imaging volume 210 (i.e., outside the "ROI" illustrated in Fig. 6). In such a scenario, the streak lines would still be detectable and could be extrapolated to a region outside the imaging volume 210 to determine their intersection.

While the Figs. 6 to 7H only show 2-dimensional imaged data slices, the steps described with reference thereto, or other steps, may be performed on 3-dimensional medical image data. In some variants, one or more of the described image data processing steps may be performed on 2-dimensional image data, and the remaining steps may be performed on 3-dimensional image data generated from the processed 2-dimensional image data.

Moreover, in case the positions of multiple markers 320a, 320b, 320c are to be determined, the method illustrated in Fig. 6 may comprise determining multiple intersections defined by the identified streak lines and associating each of these intersections with a respective one of the multiple markers 320a, 320b, 320c. As an example, intersections that are close to each other (e.g., within a predefined distance threshold) may be associated with, e.g., grouped for, an individual one of the markers 320a, 320b, 320c. Then, in step 620, a respective position in the first coordinate system 201 for each of the multiple markers 320a, 320b, 320c associated with one of the intersection groups may be determined (as described above with for an individual marker with reference to Figs. 6 to 7H).

After in step 620 the marker positions in the image coordinate system 201 have been determined for one or more of the markers 320a, 320b, 320c not included in the imaging volume 210 (conventional marker position determination techniques can be used for any of the markers 320a, 320b, 320c included in the imaging volume 210), the resulting marker positions may be exploited for automated image registration purposes. In more detail, the medical image data may be registered relative to the "live" tracking image data acquired by the tracking camera 400. If the radiopaque marker portion 324 is co-located with the optically detectable marker portion 322 as illustrated in Fig. 2A, image registration is straight forward once the positions of the same three or more markers 320a to 320f (see Figs. 2B and 6) have been determined both in the coordinate system 201 of the medical image data and in the coordinate system of the tracking image data (conventional image processing techniques can be used to identify markers in the tracking image data and to determine their positions). If other registration devices are used with radiopaque markers that are spaced apart from the optically detectable markers, image registration additionally requires knowledge of a predefined spatial relationship between these two marker types.

After the image registration has been performed, the medical image data (e.g., an origin of the image coordinate system 201) can be rooted in the patient tracker 310. The rooting may be such that any movement of the patient 300 results in a synchronous dynamic visualization of the medical image data by the display 520. The image registration also allows to visualize the current location of the tracked surgical tool 350 relative to the medical image data. Further still, the image registration may be exploited for surgical navigation. Based on planning data pre- or intra-operatively defined with respect to the medical image data (e.g., based on a target position or target trajectory for the surgical tool 350), the relationship between the current position or orientation of the surgical tool 350 and its target position or orientation may be visualized as navigation instructions on the display 520 (e.g., superimposed on the medical image data). In other implementations, the navigation instructions may be used to control a surgical robot or to generate haptic feedback for a surgeon.

As has become apparent form the above description of exemplary embodiments, the technique presented herein determines marker positions based on streak lines caused by the markers as artifacts in medical image data. While such artifacts are generally undesired, it is proposed here to exploit the streak lines as beneficial auxiliary position information. The resulting advantages are many. In some variants, non-invasive skin-placed markers can be used in combination with small imagining volumes or obese patients. In such or other variants, the marker positions determined in a conventional manner can be verified or determined more accurately based on the auxiliary position information derived from the streak lines.

The features described in relation to the exemplary embodiments shown in the drawings can be readily combined with further features described herein. It is apparent, therefore, that the present disclosure may be varied in many ways. Such variations are not to be regarded as a departure from the scope of the invention as defined by the claims appended hereto.

## Claims

1. A computer-implemented method (600) for determining a position of a marker (320a, 320b, 320c) in a first coordinate system (201) defined by medical image data generated using a radiation-based medical imaging technique and indicative of streak lines due to the marker (320a, 320b, 320c) being located in a radiation beam path, the method comprising:
identifying (610) the streak lines in the medical image data; and
determining (620), based on the identified streak lines, a position of the marker (320a, 320b, 320c) in the first coordinate system (201).

2. The computer-implemented method (600) of claim 1, wherein
the medical image data has been generated by a medical imaging device (200) defining an imaging volume (210), and wherein the marker (320a, 320b, 320c) is located outside the imaging volume (210).

3. The computer implemented method (600) according to claim 1 or 2, wherein the medical image data comprise:
i) one or more 2-dimensional image data slices (700); or
ii) 3-dimensional image data, and the method further comprises generating one or more 2-dimensional image data slices (700) from the 3-dimensional image data.

4. The computer implemented method (600) according to any of the preceding claims, wherein
determining the position of the marker (320a, 320b, 320c) comprises determining a respective intersection defined by the identified streak lines, optionally, when depending on claim 3, for each of the one or more 2-dimensional image data slices (700).

5. The computer implemented method (600) according to claim 4, further comprising:
determining the position of the marker (320a, 320b, 320c) in the first coordinate system (201) based on the determined one or more intersections and predetermined information indicative of at least one of
i) a dedicated position of each of the one or more 2-dimensional image data slices (700) in the first coordinate system (201);
ii) a dedicated orientation of each of the one or more 2-dimensional image data slices (700) in the first coordinate system (201); and
iii) dedicated pixel spacings of each of the one or more 2-dimensional image data slices (700).

6. The computer implemented method (600) according to claim 5, wherein the predetermined information comprises metadata associated with the one or more 2-dimensional image data slices (700), wherein, optionally, the metadata comprises a dedicated slice number for each of the one or more 2-dimensional image data slices (700), and wherein, further optionally, each dedicated slice number is associated with two or more of i) the dedicated position of each of the one or more 2-dimensional image data slices (700) in the first coordinate system (201); ii) the dedicated orientation of a dedicated one of the 2-dimensional image data slices (700) in the first coordinate system (201); and iii) the dedicated pixel spacings of the dedicated one of the one or more 2-dimensional image data slices (700).

7. The computer implemented method (600) according to claim 4, wherein the one or more 2-dimensional image data slices (700) comprise multiple 2-dimensional image data slices (700), the method (600) further comprising:
generating a 3-dimensional model from the multiple 2-dimensional image data slices (700) with the respective determined intersections; and
determining, based on the generated 3-dimensional model, the position of the marker (320a, 320b, 320c) in the first coordinate system (201).

8. The computer implemented method (600) according to any preceding claim, wherein multiple markers (320a, 320b, 320c) are located in the radiation beam path, the method (600) comprising:
determining multiple intersections defined by the identified streak lines;
associating each of the intersections with a respective one of the multiple markers (320a, 320b, 320c); and
determining a respective position in the first coordinate system (201) for each of the multiple markers (320a, 320b, 320c) associated with one of the intersections; and, optionally,
defining groups of identified streak lines, each group being associated with a respective one of the multiple markers.

9. The computer implemented method (600) according to claim 8, wherein the respective position in the first coordinate system (201) has been determined for at least three of the multiple markers (320a, 320b, 320c), the method further comprising:
receiving tracking data indicative of positions of the at least three of the multiple markers (320a, 320b, 320c), or of further markers having a predetermined spatial relationship relative to the at least three of the multiple markers (320a, 320b, 320c), in a second coordinate system; and
determining, based on the determined positions of the at least three of the multiple markers (320a, 320b, 320c) in the first coordinate system (201) and the tracking data, a registration between the first coordinate system (201) and the second coordinate system, and, optionally, wherein
the at least three of the multiple markers (320a, 320b, 320c) or the further markers are optically or electromagnetically detectable, and wherein the tracking data are tracking image data generated by a tracking camera (400) or an electromagnetic field sensor.

10. The computer implemented method (600) according to claim 8 or 9, further comprising:
defining a tracker based on multiple of the determined marker positions in the first or second coordinate system.

11. The computer implemented method (600) according to any preceding claim, wherein
the medical image data include Computer Tomography, CT, image data; and/or wherein
the method (600) is implemented to use machine learning for executing one or more of the method steps.

12. The computer implemented method (600) according to any preceding claim, further comprising:
processing the medical image data to facilitate at least one of the determination of the marker position and the identification of the streak lines; and, optionally, wherein
processing the medical image data comprises enhancing a contrast of the image data to improve detectability of the streak lines.

13. The computer implemented method (600) according to claim 12,
wherein
processing the medical image data comprises filtering the image data to reduce noise therein.

14. The computer implemented method (600) according to claim 12 or 13, wherein processing the medical image data comprises:
determining pixel intensity gradients; and
identifying the streak lines based on the pixel intensity gradients; and, optionally, wherein
identifying the streak lines further comprises:
defining intensity vectors based on the intensity gradients; and
grouping the intensity vectors based on their orientation and position.

15. The computer implemented method (600) according to claim 14, further comprising at least one of:
i) filtering the processed medical image data based on an estimated location of the marker (320a, 320b, 320c) relative to an object (300) that is shown in the medical image data;
ii) determining a streak line density; and
iii) determining clusters of grouped intensity vectors and determining a centre of mass of the clusters.

16. The computer implemented method (600) according to any of claims 12 to 15, wherein processing the medical image data comprises:
highlighting regions in the image data based on a degree of anisotropy.

17. A computer program product, comprising instructions that, when executed on at least one processor, cause the at least one processor to carry out the method (600) according to any of claims 1 to 16.

18. An apparatus (500) for determining a position of a marker (320a, 320b, 320c) in a first coordinate system (201) defined by medical image data generated using a radiation-based medical imaging technique and indicative of streak lines due to the marker (320a, 320b, 320c) being located in a radiation beam path and between an emitter (202), the apparatus (500) comprising a processor (510) configured to:
identify the streak lines in the medical image data; and
determine, based on the identified streak lines, a position of the marker (320a, 320b, 320c) in the first coordinate system (201).

19. The apparatus (500) according to claim 18, wherein the apparatus is configured to perform the method (600) according to any of claims 2 to 15.

20. A system (100) comprising:
the apparatus (500) according to claim 18 or 19; and
at least one of:
i) a medical imaging device (200) configured to generate medical image data using a radiation-based medical imaging technique; and
ii) a registration device with one of (1) the marker (320a, 320b, 320c), wherein the marker (320a, 320b, 320c) comprises an optically detectable portion (322) and a radiopaque portion (324) and (2) the marker (320a, 320b, 320c), wherein the marker (320a, 320b, 320b) is radiopaque, and another marker that is optically or electromagnetically detectable and has a predefined spatial relationship to the radiopaque marker (320a, 320b, 320c).
